# EUROPEAN PATENT APPLICATION

(11) **EP 0 972 839 A1**
(43) Date of publication of application: **19.01.2000**
(21) Application number: 99110251.8
(22) Date of filing: 02.06.1992
(51) Int. Cl.: C12N 15/74, A61K 39/04, C12N 1/21

(54) **Insertional mutations in mycobacteria**

(30) Priority: 13.06.1991 US 714656; 12.12.1991 US 806706
(62) Divisional of application: 92913432.8
(71) Applicant: ALBERT EINSTEIN COLLEGE OF MEDICINE OF YESHIVA UNIVERSITY, a division of YESHIVA UNIVERSITY, Bronx New York 10461 (US)
(72) Inventor: Jacobs, William R., City Island, New York 10464 (US); Bloom, Barry, Hastings-on-Hudson, New York 10706 (US); Kalpana, Ganjam V., Yonkers, New York 10710 (US); Cirillo, Jeffrey D., Bronx, New York 10461 (US)
(74) Representative: LOUIS, PÖHLAU, LOHRENTZ & SEGETH

(57) **Abstract**

A mutated mycobacterium selected from the class consisting of mutated M.bovis-BCG, mutated M.tuberculosis, and mutated M.leprae. The mutation of M.bovis-BCG, M.tuberculosis, or M.leprae is preferably effected through an insertional mutation of a mycobacterial gene. The insertional mutagenesis may be effected, for example, through illegitimate recombination or by a mycobacterial transposon. Such mutated mycobacteria may then be transformed with an expression vector(s) containing a complement gene to the gene which is mutated, and preferably also including a heterologous gene.

## Description

This application is a continuation-in-part of application Serial No. 714,656, filed June 13, 1991.

This invention relates to mutagenesis of mycobacteria. More particularly, this invention relates to the generation of insertional mutations in mycobacteria.

Certain mycobacteria represent major pathogens of man and animals. For example, tuberculosis is generally caused in humans by Mycobacterium tuberculosis, and in cattle by Mycobacterium bovis, which may also be transmitted to humans and other animals. Mycobacteria leprae is the causative agent of leprosy. M. tuberculosis and mycobacteria of the avium-intracellulare-scrofulaceum group (MAIS group) represent major opportunistic pathogens of patients with acquired immune deficiency syndrome (AIDS). M. pseudotuberculosis is a major pathogen of cattle.

On the other hand, Bacille Calmette-Guerin, or BCG, an avirulent strain of M. bovis, is widely used in human vaccines, and in particular is used as a live vaccine, which is protective against tuberculosis. BCG is the only childhood vaccine which is currently given at birth, has a very low incidence of adverse effects, and can be used repeatedly in an individual (e.g., in multiple forms). In addition, BCG and other mycobacteria (e.g., M. smegmatis), employed in vaccines, have adjuvant properties among the best currently known and, therefore, stimulate a recipient's immune system to respond to antigens with great effectiveness.

It has been suggested by Jacobs, et at, Nature, Vol. 327, No. 6122, pgs. 532-535 (June 11, 1987) that BCG could be used as a host for the construction of recombinant vaccines. In other words, it was suggested to take an existing vaccine (in this case against tuberculosis) and expand its protective repetoire through the introduction of one or more genes from other pathogens. Because BCG vaccines are administered as live bacteria, it is essential that any foreign antigens, polypeptides, or proteins expressed by the bacteria are not lost from the bacteria subsequent to vaccination.

Transformation, the process whereby naked DNA is introduced into bacterial cells, has been carried out successfully in mycobacteria. Jacobs, et al (1987), hereinabove cited, have described transformation of mycobacteria through chemical methods, and Snapper, et al. PNAS, Vol. 85, pgs. 6987-6991 (September 1988) have described transformation of mycobacteria by electroporation. Electroporation can give from 10⁵ to 10⁶ transformants per µg of plasmid DNA and such plasmid DNA's may carry genes for resistance to antibiotic markers such as kanamycin (Snapper, et al 1988) to allow for selection of transformed cells from non-transformed cells.

Jacobs, et al (1987) and Snapper, et al (1988) have also described the use of cloning vehicles, such as plasmids and bacteriophages, for carrying genes of interest into mycobacteria.

Combination of the above-mentioned techniques, along with standard tools of molecular cloning (e.g., use of restriction enzymes, etc.) allows the cloning of genes of interest into vectors and introduction of such genes into mycobacteria. To express these genes, it is important to have available signals for gene expression, in particular, transcription promoter elements. Such promoter elements have been isolated from mycobacterial heat shock genes, and have been used to express foreign antigens in mycobacteria.

Molecular genetics of mycobacteria, however has only recently begun to be developed, in part because mycobacteria present formidable obstacles to genetic study in that mycobacteria, in general, clump in culture and grow very slowly. The direct selection of mutants by employing transposons (also known as random insertional mutagenesis) has been a useful approach to the mutational analysis of microbial pathogenesis (Isberg, et al., Curr. Top. Microbiol. Immunol., Vol. 118, pgs. 1-11 (1985); Taylor, et al., J. Bacteriol, Vol. 171, pgs. 1870-1878 (1989); Fields, et al., Science, Vol. 243, pgs. 1059-1061 (1989); Bernardini, et al., Proc. Nat. Acad. Sci., Vol. 86, pgs. 3867-3871 (1989)); such selection of mutants, however, had not been described in mycobacteria.

Objects of the present invention include the generation of mutations in mycobacteria and/or the introduction of heterologous genes into mycobacteria; in particular, the generation of mutations of mycobacteria employed in vaccines, such as BCG, as well as the generation of mutations in pathogenic mycobacteria, such as M. tuberculosis or M. leprae, whereby such mutations make the mycobacteria non-pathogenic. Heterologous genes which may be introduced into the mycobacteria include, but are not limited to, genes for protective antigen(s) for a variety of pathogens, and/or for other therapeutic agents.

In accordance with an aspect of the present invention, there is provided a mutated mycobacterium selected from the class consiting of mutated M.bovis- BCG, mutated M. tuberculosis, and mutated M. leprae. The term "mutated M.bovis- BCG", or "mutated BCG", or "mutated M. tuberculosis", or or "mutated M. leprae" as used herein means that the M.bovis - BCG, M.tuberculosis, or M. leprae includes at least one mutated gene such that the expression or the function of the gene is varied with respect to the non-mutated gene in the parent strain.

Preferably, the mycobacterium is mutated through an insertional mutation of a mycobacterial gene. The insertional mutation of the mycobacterial gene may be effected through illegitimate recombination of DNA into the mycobacterial chromosome, or by homologous recombination, or by the insertion of a mycobacterial transposon into a mycobacterial gene. Preferably the insertional mutation is effected by illegitimate recombination of DNA into the mycobacterial chromosome.

In accordance with another aspect of the present invention, there is provided a mutated M. bovis which is mutated through an insertional mutation of an M. bovis gene. The insertional mutation of the M. bovis gene may be effected as hereinabove described for mutated M. bovis-BCG, mutated M. tuberculosis, and mutated M. leprae.

The DNA which is integrated into the mycobacterial chromosome through illegitimate recombination may be a linear DNA fragment or may be a circular DNA. Preferably, the DNA is a linear DNA fragment.

Applicants have found that illegitimate recombination, which is a rare phenomenom in prokaryotes, may be effected in M.bovis-BCG and M. tuberculosis by transforming BCG or M. tuberculosis with a linearized plasmid. Transformation may be accomplished by any means known to those skilled in the art, such as, for example, electroporation, or by the generation of protoplasts into which the transforming DNA is inserted, followed by regeneration of the cell wall, as described in Jacobs (1987) and Snapper (1988).

In one embodiment, the DNA which is integrated into the mycobacterium chromosome through illegitimate recombination includes a selectable marker. Selectable markers which may be employed included a kanamycin resistance marker, a bleomycin resistance marker, or a hygromycin resistance marker, or a bacteriophage resistance marker, such as, but not limited to, mycobacteriophage L5, L1, Bxb1, Bxb2, Bxb3, D29, or TM4 resistance markers.

In another embodiment, the DNA includes at least one DNA sequence which encodes a protein heterologous to mycobacteria.

The at least one DNA sequence which encodes a protein heterologous to mycobacteria may be DNA which is all or a portion of a gene encoding protein(s) or polypeptide(s) of interest.

Proteins or polypeptides of interest, which may be encoded by the at least one DNA sequence include, but are not limited to, antigens, anti-tumor agents, enzymes, lymphokines, pharmacologic agents, immunopotentiators, and reporter molecules of interest in a diagnostic context.

Antigens for which the at least one DNA sequence may encode include, but are not limited to, Mycobacterium leprae antigens; Mycobacterium tuberculosis antigens; Rickettsia antigens; malaria sporozoites and merozoites; diphtheria toxoids; tetanus toxoids; Clostridium antigens; Leishmania antigens; Salmonella antigens; Borrelia antigens; Mycobacterium africanum antigens; Mycobacterium intracellulare antigens; Mycobacterium avium antigens; Treponema antigens; Pertussis antigens; Schistosoma antigens; Filaria antigens; Herpes virus antigens; influenza and parainfluenza virus antigens; measles virus antigens; mumps virus antigens; hepatitis virus antigens; Shigella antigens; Neisseria antigens; rabies antigens, polio virus antigens; Rift Valley Fever virus antigens; dengue virus antigens; Human Immunodeficiency Virus (HIV) antigens; respiratory syncytial virus (RSV) antigens; snake venom antigens; and Vibrio cholera antigens. Enzymes which may be encoded include, but are not limited to, steroid enzymes.

Anti-tumor agents which may be encoded by the at least one DNA sequence include, but are not limited to, interferon-α, interferon-β, or interferon-γ, and tumor necrosis factor, or TNF. Lymphokines which may be encoded include, but are not limited to, interleukins 1 through 8.

Reporter molecules which may be encoded include, but are not limited to, luciferase, β-galactosidase, β-glucuronidase, and catechol dehydrogenase.

Other peptides or proteins which may be encoded by the at least one DNA sequence include, but are not limited to, those which encode for stress proteins, which can be administered to evoke an immune response or to induce tolerance in an autoimmune disease (e.g., rheumatoid arthritis).

In one embodiment the DNA which integrates into the mycobacterial chromosome through illegitimate recomination may be derived from a plasmid. The plasmid may be a shuttle plasmid which includes a bacterial origin of replication such as an E.coli origin of replication, a Bacillus origin of replication, a Staphylococcus origin of replication, a Streptomyces origin of replication, or a pneumococcal origin of replication. The plasmid, in another embodiment, may include a mycobacterial origin of replication, or may be a shuttle plasmid including a mycobacterial origin of replication plus a bacterial origin of replication as hereinabove described. Preferably, the plasmid is linearized prior to integration into the mycobacterial chromosome through illegitimate recombination.

In a preferred embodiment, a linear DNA, such as, for example, a linearized plasmid, is integrated into an M. bovis chromosome, an M.bovis-BCG chromosome or an M.tuberculosis chromosome, or an M. leprae chromosome, such as, for example, a BCG chromosome, through illegitimate recombination. The DNA, through such integration, will cause an insertional mutation of a gene(s) in the mycobacterial chromosome. For example the DNA may cause an insertional mutation of gene encoding an enzyme which is essential in a biosynthetic pathway of a nutrient or an amino acid. The transformed mycobacteria are then screened in order to determine the gene(s) which is mutated. For example, the mutated mycobacteria may be grown on minimal media (without amino acids) and on media containing various amino acids to determine the nutritional requirements of the mutated mycobacteria; i.e., the mutated mycobacteria may be screened for auxotrophy. Once the mutated gene is identified, the complement (i.e., non-mutated) gene is isolated and cloned into an expression vector. The expression vector is then transformed into the mutated mycobacterium, whereby the complement gene is expressed in the mutated mycobacterium, and the mutated mycobacterium becomes prototrophic. The expression vector may also include a gene encoding for protein or polypeptide heterologous to the mutated mycobacterium. Such proteins or polypeptides may be those which are hereinabove described. Selection for mutated mycobacteria containing the complement gene and a gene encoding for a heterologous protein or polypeptide may thus be based on the ability of the mutated mycobacteria, into which the complement and the heterologous genes are introduced, to survive, when such mutated mycobacteria are grown on appropriate media.

In one embodiment, the expression vector is a DNA which comprises a first DNA sequence which is a phage DNA portion encoding bacteriophage integration into a mutated mycobacterium chromosome, and a second DNA sequence which encodes the complement gene, and may further include a third DNA sequence encoding at least one protein or polypeptide which is heterologous to the mutated mycobacterium in which the DNA is to be integrated.

The term "phage DNA portion," as used herein means that the DNA sequence is derived from a phage and lacks the DNA which is required for phage replication.

Bacteriophages from which the phage DNA portion may be derived include, but are not limited to, mycobacteriophages, such as but not limited to the L5, L1, Bxb1, Bxb2, Bxb3, D29, and TM4 mycobacteriophages; the lambda phage of E.coli; the toxin phages of Corynebacteria; phages of Actinomyces and Norcadia, the ØC31 phage of Streptomyces; and the P22 phage of Salmonella. Preferably, the phage DNA portion encodes mycobacteriophage integration into a mycobacterium chromosome.

Preferably, the first DNA sequence includes DNA encoding integrase, which is a protein that provides for integration of the DNA into the mutated M. bovis or mutated M.bovis-BCG or mutated M. tuberculosis or mutated M. leprae mycobacterial chromosome. Most preferably, the first DNA sequence also includes DNA which encodes an attP site.

The DNA sequence encoding the attP site, and the integrase provides for an integration event which is referred to as site-specific integration. DNA containing the attP-site and the integrase gene is capable of integration into a corresponding attB site of an M.bovis-BCG, M.tuberculosis, or M. leprae chromosome.

It is to be understood that the exact DNA sequence encoding the attP site may vary among different phages, and that the exact DNA sequence encoding the attB site may vary among M. bovis, M. bovis-ECG, M.tuberculosis, and M. leprae.

The integration event results in the formation of two new junction sites called attL and attR, each of which contain part of each of attP and attB. The inserted and integrated non-phage DNA which includes the first, second, and preferably third DNA sequences, is flanked by the attL and attR sites. The insertion and integration of the phage DNA portion results in the formation of a transformed mutated mycobacterium which includes an insertionally mutated mycobacterial gene, a complement gene, and preferably a DNA sequence which encodes a protein or polypeptide which is heterologous to mycobacteria.

The third DNA sequence which encodes a protein or polypeptide heterologous to mycobacteria may be DNA which is all or a portion of a gene encoding protein(s) or polypeptide(s) of interest, such as those hereinabove described; DNA encoding a selectable marker or markers; or DNA encoding both a selectable marker or markers and at least one protein or polypeptide of interest.

Selectable markers which may be encoded include, but are not limited to, the kanamycin resistance marker, the neomycin resistance marker, the chloramphenicol resistance marker, the hygromycin resistance marker, or a bacteriophage resistance marker, such as those hereinabove described.

The phage DNA portion, which includes the first DNA sequence encoding mycobacterium phage integration, and the second DNA sequence encoding the complement gene, and the third DNA sequence, if present, encoding at least one protein or polypeptide heterologous to mycobacteria, may be constructed through genetic engineering techniques known to those skilled in the art. In one embodiment, the phage DNA portion may be a plasmid including, in addition to the DNA encoding integration, the DNA encoding the complement gene, and, if present, the DNA encoding a heterologous protein, an origin of replication for any of a wide variety of organisms which includes, but is not limited to, E.coli, Streptomyces species, Bacillus species, Staphylococcus species, Shigella species, Salmonella species, and various species of pneumococci. Preferably the plasmid includes an origin of replication for E.coli.

The phage DNA portion also may include a suitable promoter. Suitable promoters include, but are not limited to, mycobacterial promoters such as the BCG HSP60 and HSP70 promoters; mycobactin promoters of M.tuberculosis and BCG, the superoxide dismutase promoter, the α-antigen promoter of M.tuberculosis and BCG, the MBP-70 promoter, the 45 kda antigen promoter of M.tuberculosis and BCG; the mycobacterial asd promoter; the mycobacterial 14 kda and 12 kda antigen promoters; mycobacteriophage promoters such as the Bxb1, Bxb2, and Bxb3 promoters the L1 and L5 promoters, the D29 promoter, and the TM4 promoters; E.coli promoters; or any other suitable promoter. The selection of a suitable promoter is deemed to be within the scope of those of ordinary skill in the art from the teachings contained herein.

The promoter sequence may, in one embodiment, be part of an expression cassette which also includes a portion of the gene normally under the control of the promoter. For example, when a mycobacterial HSP60 or HSP70 promoter is employed, the expression cassette may include, in addition to the promoter, a portion of the gene for the HSP60 or HSP70 protein. When the expression cassette and the DNA encoding a heterologous protein or polypeptide are expressed, the protein expressed by the cassette and the DNA encoding the heterologous protein or polypeptide is a fusion protein of a fragment of a mycobacterial protein (e.g., the HSP60 or HSP70 protein), and of the heterologous protein.

Preferably, the transcription initiation site, the ribosomal binding site, and the start codon, which provides for the initiation of the translation of mRNA, are each of mycobacterial origin. The stop codon, which stops translation of mRNA, thereby terminating the synthesis of the complement protein and/or the heterologous protein, and the transcription termination site, may be of mycobacterial origin, or of other bacterial origin, or such stop codon and transcription termination site may be those of the DNA encoding the complement gene or DNA encoding the heterologous protein or polypeptide, when such DNA is present.

Such DNA which includes a first DNA sequence which is a phage DNA portion encoding bacteriophage integration into a mycobacterium chromosome, and a second DNA sequence encoding a complement gene, and if present, a third DNA sequence encoding at least one protein or polypeptide which is heterologous to the mutated mycobacterium in which the DNA is to be integrated is further described in Application Serial No. 553,907, filed July 16,1990.

In another embodiment, the expression vector includes DNA which encodes the complement gene, and may further include DNA which encodes a protein or polypeptide heterologous to the mutated mycobacterium which expresses the protein or polypeptide, such as those hereinabove described, and a promoter selected from the class consisting of mycobacterial promoters and mycobacteriophage promoters for controlling expression of the DNA encoding the complement gene and, if present, the DNA encoding the heterologous protein or polypeptide. The mycobacterial promoters may be those as hereinabove described with respect to the DNA which encodes phage integration into a mycobacterium chromosome. The promoter may also be part of an expression cassette which also includes a portion of the gene normally under the control of the promoter, as hereinabove described. Also, the transcription initiation codon, the ribosomal binding site, and the start codon, may each be of mycobacterial origin, and the stop codon, and the transcription termination site, may be of mycobacterial origin, or of other bacterial origin, or the stop codon and transcription termination site may be those of the DNA encoding the complement gene, or of the DNA encoding the heterologous protein or polypeptide, also as hereinabove described.

In one embodiment, the mycobacterial promoter is a BCG promoter.

In another embodiment, the heterologous protein or polypeptide may be a selectable marker. Such selectable markers include, but are not limited to, the β-galactosidase marker, the kanamycin resistance marker, the chloramphenicol resistance marker, the neomycin resistance marker, the hygromycin resistance marker, or a bacteriophage resistance marker such as those hereinabove described.

In one embodiment, the expression vector further includes a mycobacterial origin of replication.

In accordance with another embodiment, such an expression vector may be a plasmid. The plasmid may be a non-shuttle plasmid, or may be a shuttle plasmid which further includes a bacterial origin of replication such as an E.coli origin of replication, a Bacillus origin of replication, a Staphylococcus origin of replication, a Streptomyces origin of replication, or a pneumococcal origin of replication.

The vector may further include a multiple cloning site, and the DNA encoding for the complement gene and/or the DNA encoding for the heterologous protein is inserted in the multiple cloning site.

Such an expression vector including a mycobacterial promoter or a mycobacteriophage promoter is further described in application Serial No. 642,017, filed January 16, 1991, which is a continuation of application Serial No. 552,828, filed July 16, 1990, now abandoned. The contents of application Serial No. 642,017 are hereby incorporated by reference.

In another embodiment, the expression vector which includes the complement gene and/or DNA encoding for a protein or polypeptide which is heterologous to mycobacteria is a shuttle phasmid vector, which replicates as a plasmid in bacteria and as a phage in mycobacteria. In one embodiment, the shuttle phasmid vector includes two species of cohesive end sites: one for lambda phage, which functions in E. coli; and one for mycobacteria (eg., the mycobacteriophage TM) which functions in mycobacteria. Preferably, such shuttle phasmid vector has a unique site (eg., a unique EcoRI sits) into which the complement gene and/or DNA encoding a protein or polypeptide heterologous to mycobacteria may be inserted. Examples of such shuttle phasmid vectors are further described in Application Serial No. 361,944, filed June 5, 1989 the contents of which are hereby incorporated by reference.

It is also to be understood that within the scope of the present invention, the complement gene may be contained in one expression vector, and a gene encoding a protein or polypeptide heterologous to mycobacteria may be contained in another expression vector.

As hereinabove stated, the mycobacterium may include an insertional mutation of a mycobacterial gene which is effected through the insertion of a mycobacterial transposon into a mycobacterial gene. Thus, in accordance with another aspect of the present invention, there is provided a mycobacterial transposon which is capable of inserting randomly into a mycobacterial chromosome to effect an insertional mutation of a mycobacterial gene. The mycobacterial transposon may, for example, insert randomly into an M. bovis chromsome, an M. bovis-BCG chromosome, an M. tuberculosis chromosome, an M. avium chromosome, an M. leprae chromosome, or an M. smegmatis chromosome. In one embodiment, the transposon is the IS1096 transposon of M. smegmatis. The IS1096 transposon, which is hereinafter described, is 2,275 bp in length, and contains two open reading frames which may encode proteins involved in transposition. Although the IS1096 transposon is found in M. smegmatis, it is contemplated that the transposon may be employed in generating insertional mutations in other species of mycobacteria, such as those hereinabove mentioned.

The term "transposon" as used herein, means a non-mutated transposon or a mutated transposon in which a portion of the transposon sequence has been deleted and/or replaced, and/or wherein the transposon contains additional DNA sequence(s).

In general, a transposon contains an inverted repeat sequence at each (5' and 3') end, and a gene(s) encoding a transposase enzyme(s) between the inverted repeat sequences. The transposase(s) acts upon the inverted repeat sequences so as to enable the transposon to remove itself from a DNA (eg., chromosomal DNA, plasmid DNA, phage DNA, etc.) and insert or transpose into another DNA, or into another region of the same DNA. In some instances, the transposon may also include gene(s) encoding resolvase(s) and/or regulatory protein(s) which regulate transposition.

The insertional mutation, which is effected by a mycobacterial transposon, may be effected by random transposition, or "hopping" of a transposon contained in a mycobacterial chromosome from one region of the chromosome into a mycobacterial gene contained in another region of the chromosome, or the transposon may be transfected into the mycobacterium by a variety of means. Subsequent to transfection, the transposon may then insert at random into the mycobacterial chromosome to effect an insertional mutation.

The transposon may be contained in any plasmid or phage DNA vector, including mycobacterial vectors and mycobacteriophage vectors. In one embodiment, the transposon may be contained in a mycobacterial vector. The vector may include a mycobacterial promoter or mycobacteriophage promoter, such as those hereinabove described. In another embodiment, the transposon may be contained within a mycobacterial expression vector or DNA which encodes phage integration into a mycobacterium chromosome, such as hereinabove described, or the transposon may be contained within phage DNA which can replicate within one organism, but not within the organism into which the phage is transfected. Alternatively, the transposon may be contained in a conjugative plasmid which can replicate in a bacterium other than a mycobacterium but cannot replicate in mycobacteria. For example, the transposon may be contained in a conjugative plasmid which can replicate in a bacterium such as E.coli or Streptomyces, but cannot replicate in M.bovis-BCG.

In one embodiment, a construct is made in which the gene(s) encoding a transposase(s) and gene(s) encoding resolvase(s) and/or regulatory protein(s) if also present, is removed from its normal position in the transposon (between the inverted repeat sequences), and is placed, in the resulting construct, outside the inverted repeat sequences. A selectable marker, such as those hereinabove described (eg., antibiotic resistance) is then placed between the inverted repeat sequences. The transposable element thus includes the inverted repeat sequences and the selectable marker. This construct is then cloned into a mycobacterial expression vector which includes a mycobacterial origin of replication and may also include a mycobacterial promoter or mycobacteriophage promoter such as those hereinabove described. The mycobacterial origin of replication of the plasmid is then mutated such that the mycobacterial origin of replication becomes temperature sensitive (eg., the plasmid can replicate at 30°C but not at 37°C). The plasmid is then transfected into mycobacteria at 30°C, and the mycobacteria are selected for antibiotic resistance. Antibiotic resistant colonies are then plated out on complete medium containing the antibiotic at 37°C. At 37°C, the plasmid containing the mutated mycobacterial origin of replication cannot replicate. Those mycobacteria which survive are those in which the transposable element containing the inverted repeat sequences and the antibiotic resistance marker has transposed from the plasmid into the mycobacterial chromosome. Because the transposase gene(s), as well as resolvase gene(s) and/or regulatory protein gene(s), if present, remains in the plasmid, the transposase gene(s) as well as resolvase and/or regulatory protein gene(s), if present, is lost upon further replication of the mycobacteria, and, therefore, the transposed construct, upon insertion into the mycobacterial chromosome, will not undergo any subsequent transpositions.

Alternatively, the construct hereinabove described may be cloned into a vector which cannot replicate in mycobacteria; i.e., the vector does not include a mycobacterial origin of replication. The vector is then transfected into mycobacteria. Because the vector containing the construct cannot replicate in mycobacteria, the vector will become lost. The mycobacteria, after transfection, are screened for antibiotic resistance. Those mycobacteria which survive are those in which the transposable element containing the inverted repeat sequences and the antibiotic resistance marker has transposed from the plasmid into the mycobacterial chromosome. The transposase gene(s), plus resolvase and/or regulatory protein gene(s), if present, remains in the plasmid, and therefore is lost upon further replication of the mycobacteria, and, therefore, the transposed construct, upon insertion into the mycobacterial chromosome, will not undergo further transpositions.

It is also contemplated that a mycobacterial transposon may be constructed which includes at least one DNA sequence which encodes a protein heterologous to mycobacteria. The at least one DNA sequence which encodes a protein heterologous to mycobacteria may be DNA which is all or a portion of a gene encoding protein(s) or polypeptide(s) of interest. The proteins or polypeptides of interest may be those hereinabove described. The at least one DNA sequence which encodes a protein heterologous to mycobacteria may be under the control of a suitable promoter. Such a transposon may be constructed by techniques known to those skilled in the art. The transposon may also include tranposase gene(s) and/or a selectable marker such as those hereinabove described. Alternatively, a construct may be formed in which the transposase gene(s), and resolvase gene(s) and/or regulatory protein gene(s) if present, is removed from a mycobacterial transposon and placed outside the inverted repeat sequences, and the at least one DNA sequence encoding a protein heterologous to the mycobacterium, and preferably a selectable marker, is placed between the inverted repeat sequences. The construct may then be placed into a vector such as hereinabove described for transfection into a mycobacterium. The transposable element, containing the inverted repeat sequences, the at least one DNA sequence encoding the protein or polypeptide heterologous to the mycobacterium, and the selectable marker, may then insert randomly into the mycobacterium chromosome.

Once the insertional mutation of a mycobacterial gene is effected by a mycobacterial transposon, the mycobacteria may be screened to determine the gene(s) which is mutated, by methods hereinabove described. Once the mutated gene is identified, the complement gene may be isolated and cloned into an expression vector which is transformed into the mutated mycobacterium.

It is also contemplated within the scope of the present invention that insertional mutations may be generated in M. tuberculosis of gene(s) conferring virulence upon M. tuberculosis, thereby transforming M. tuberculosis from a pathogenic to a non-pathogenic organism. Such mutated non-pathogenic M. tuberculosis organisms may be employed in a vaccine for protection against tuberculosis. If desired, the mutated non-pathogenic M. tuberculosis organisms may be subjected to further insertional mutagenesis, as hereinabove described, whereby auxotrophy may be conferred upon the mutated M. tuberculosis organisms. Such mutated M. tuberculosis organisms may then be genetically engineered through techniques such as those hereinabove described, so as to express the complement gene and/or genes for heterologous proteins of interest, such as those hereinabove described.

The mutated mycobacteria hereinabove described, which are transformed with DNA which encodes a protein(s) or polypeptide(s) heterologous to mycobacteria may be employed in the production of a vaccine or therapeutic agent, depending upon the protein(s) or polypeptide(s) expressed by the transformed mutated mycobacteria.

To form such a vaccine or therapeutic agent, the transformed mutated mycobacteria are administered in conjunction with a suitable pharmaceutical carrier. As representative examples of suitable carriers there may be mentioned: mineral oil, alum, synthetic polymers, etc. Vehicles for vaccines and therapeutic agents are well known in the art and the selection of a suitable vehicle is deemed to be within the scope of those skilled in the art from the teachings contained herein. The selection of a suitable vehicle is also dependent upon the manner in which the vaccine or therapeutic agent is to be administered. The vaccine or therapeutic agent may be in the form of an injectable dose and may be administered intramuscularly, intravenously, orally, intradermally, or by subcutaneous administration.

Other means for administering the vaccine or therapeutic agent should be apparent to those skilled in the art from the teachings herein; accordingly, the scope of the invention is not to be limited to a particular delivery form.

When the transformed mutated mycobacteria are employed as a vaccine, such a vaccine has important advantages over other presently available vaccines. Mycobacteria have, as hereinabove indicated, adjuvant properties among the best currently known and, therefore, stimulate a recipient's immune system to respond with great effectiveness. This aspect of the vaccine induces cell-mediated immunity and thus is especially useful in providing immunity against pathogens in cases where cell-mediated immunity appears to be critical for resistance. Also, mycobacteria may stimulate long-term memory or immunity. It thus may be possible to prime long-lasting T cell memory, which stimulates secondary antibody responses neutralizing to the infectious agent or the toxin. Such priming of T cell memory is useful, for example, against tetanus and diphtheria toxins, pertussis, malaria, influenza virus, Herpes virus, rabies, Rift Valley Fever virus, dengue virus, measles virus, Human Immunodeficiency Virus (HIV), respiratory syncytial virus, human tumors, and snake venoms. Another advantage in employing mycobacteria transformed in accordance with the present invention as a vaccine or a therapeutic agent is that mycobacteria in general have a large genome (i.e., approximately 3 x 10⁶ base pairs in length). Because the genome is large, it is able to accommodate a large amount of DNA from other source(s), and may possibly be employed to make a vaccine and/or therapeutic agent containing DNA sequences encoding more than one antigen and/or therapeutic agent.

The invention will now be described with respect to the following examples; however, the scope of the present invention is not intended to be limited thereby.

### EXAMPLE 1 (Comparative)

### Random Shuttle Mutagenesis of M. smegmatis

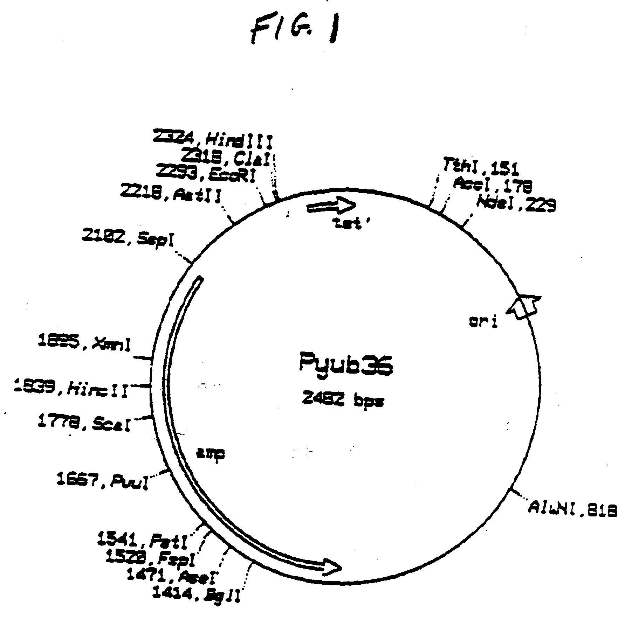

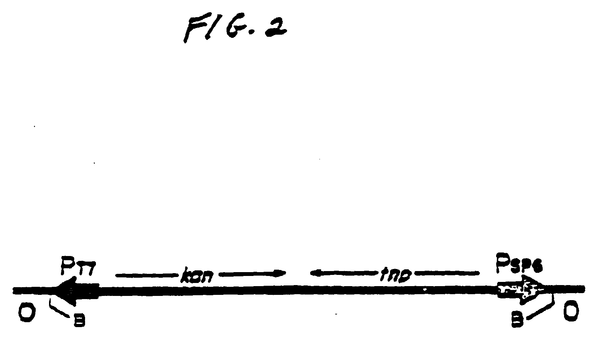

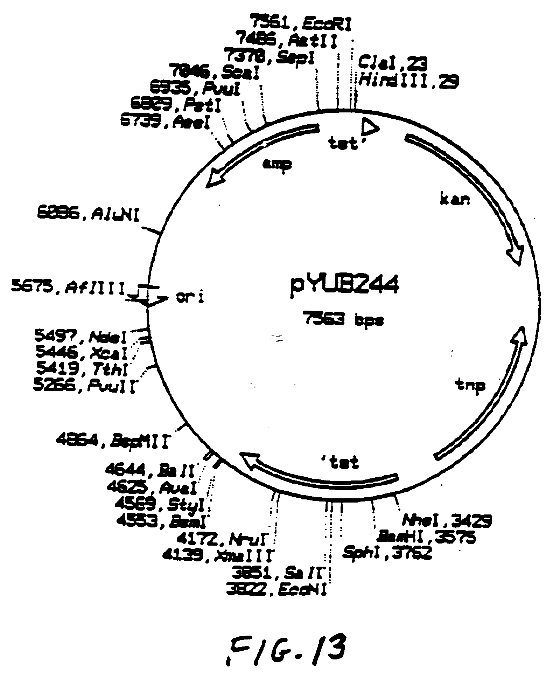

Chromosomal DNA of M. smegmatis strain mc²6 (Jacobs, Jr., et al. Nature, Vol. 327, pgs. 532-536 (1987)) was partially digested with Map I. Following size selection, DNA inserts of 4kb to 7kb were ligated to Cla I-digested pYUB36 (Figure 1). pYUB36 includes a gene (amp) for ampicillin resistance, and an E. coli origin of replication, and is a derivative of pBR 322 (Bolivar, et al., Gene, Vol. 2, pgs. 95-113 (1977) in which a nonessential 1.9kb EcoRV to Pvu II fragment has been deleted. After the DNA inserts have been ligated to ClaI-digested pYUB36, the resulting constructs are transformed into E. coli strain ec² 270, a derivative of E. coli strain X 2338 (Jacobs, et al., Proc. Natl. Acad. Sci., Vol. 83, pgs. 1926-1930 (1986)) into which transposon Tn5 seq 1 (Nag., et al., Gene, Vol. 64, pgs. 135-145 (1988)) (Figure 2) is inserted at an unknown location of the chromosome. Tn5 Seq 1 encodes the neo gene which confers kanamycin resistance to both E. coli and mycobacteria (Snapper, et at., Proc. Natl. Acad. Sci., Vol. 85, pgs. 6987-6991 (1988)), permits selection of insertions into DNA sequences cloned into plasmid vectors using its neomycin hyper-resistance phenotype (Berg, et al., Genetics, Vol. 105, pgs. 813-828 (1983)), and Tn5seq1 lacks the cryptic gene present in Tn5 which encodes streptomycin resistance, which is an important biohazard consideration for the genetic engineering of M. tuberculosis strains as hereinafter described. (U.S. Fed. Register, Vol. 51, pg. 16957). The transformed E. coli organisms are plated on L-agar medium containing both ampicillin and kanamycin at concentrations of 40 µg/ml and 50 µg/ml, respectively. About 30,000 individual transformants were pooled and samples were diluted 10⁻³ into 20 independent 5 ml cultures and incubated at 37°C overnight. A 200 µl sample from each overnight culture yielded approximately 1000 colonies on L-agar containing 250 µg/ml neomycin, whereby such colonies were selected for neomycin hyper-resistance, which is conferred through transposition of Tn5 seq 1 from the E. coli chromosome into the transfecting plasmids. Plasmid DNA from the neomycin hyper-resistant colonies was isolated as described in Birnboim, Methods in Enzymology, Vol. 100, pgs 243-255 (1983), and was re-transformed into E. coli strain X2338, and plasmid DNA was subsequently isolated and prepared from the transformants. The Tn5 seq 1-mutagenized plasmid library was then electroporated into either M. smegmatis strain mc²6 or M. smegmatis strain mc²155 (Snapper, at al., Mol. Microbiol., Vol. 4, pgs. 1911-1919 (1990)), and kanamycin-resistant transformants were selected on K-agar (Middlebrook 7H10 agar supplemented with 5 mg/ml casamino acids (Difco), 100 µg/ml diaminopimelic acid, 50 µg/ml thymidine, 40 µg/ml uracil, 133 µg/ml adenosine, 0.2% glycerol, albumen-dextrose complex, and 10 µg/ml cyclohexamide) containing 20 µg/ml kanamycin. Kanamycin resistant transformants were obtained at a frequency of 20 to 40 per µg of plasmid DNA. No kanamycin resistant colonies were obtained with a control pBR322::Tn5seq 1 plasmid (also known as pYUB244, as shown in Figure 13) which lacked homologous DNA sequences. About 800 individual M. smegmatis transformants were screened for auxotrophy by streaking onto minimal Sauton medium without asparagine. The transformants were screened for auxotrophy as described in Davis, et al., A Manual for Genetic Engineering: Advanced Bacterial Genetics, Cold Spring Harbor Laboratory (1980), Appendix 2, pgs. 209-210. Three auxotrophs were obtained from this screen and their nutritional requirements were determined by plating on Sauton agar plates supplemented with one or the other of 11 pools of nutrients used for the auxonography analysis of E. coli as described in Davis, et al. (1980). The auxotrophs obtained by this screen were a methionine-requiring auxotroph (M. smegmatis strain mc²311), a pyridoxine-requiring auxotroph (M. smegmatis strain mc²313), and an auxotroph which is incompletely characterized.

### EXAMPLE 2

### A. Isolation of BCG Pasteur Complementing clones of M. smegmatis methionine auxotroph

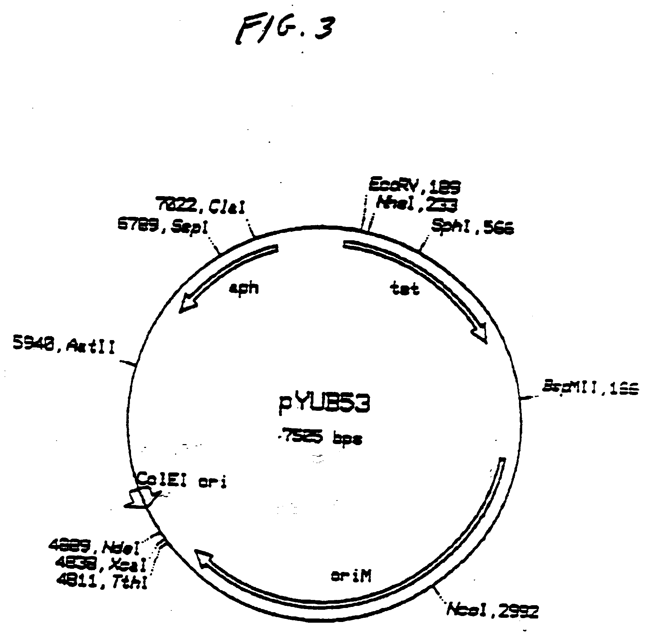

A genomic library of BCG Pasteur (obtained from the WHO BCG Reference Laboratory at the Statens Seruminstitut in Copenhagen) was constructed by ligating 4kb-7kb Msp-I digested chromosomal DNA fragments to ClaI-digested pYUB53 (Figure 3), an E. coli/mycobacteria shuttle vector including an E. coli origin of replication and a mycobacterial origin of replication. Ligated DNA's were then introduced into E. coli strain ec²270, and plasmids isolated from the pool of E. coli as described in Snapper, et al. (1988), were electroporated into the M. smegmatis methionine auxotroph, mc²311. Such plasmids, because they include a mycobacterial origin of replication, do not integrate into the mycobacterial chromosome, but self-replicate within the mycobacterium. Plasmids which conferred prototrophy to mc²311 (referred to as met-complementing clones) were isolated as described in Snapper, et al. (1988)

### B. Inactivation of met-complementing clones.

The met-complementing clone, named pYUB121, underwent insertional inactivation in E. coli by neomycin hyper-resistance selection as hereinabove described in Example 1. Individual pYUB121 clones were then screened for the loss of their ability to complement the methionine auxotrophic mutation of mc²311 according to the procedure described in Davis, et al. (1980).

### C. Recombination in BCG.

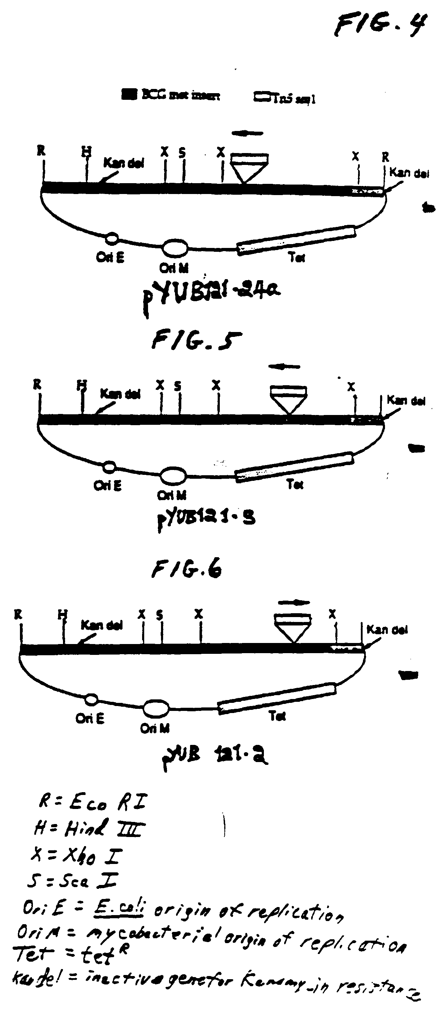

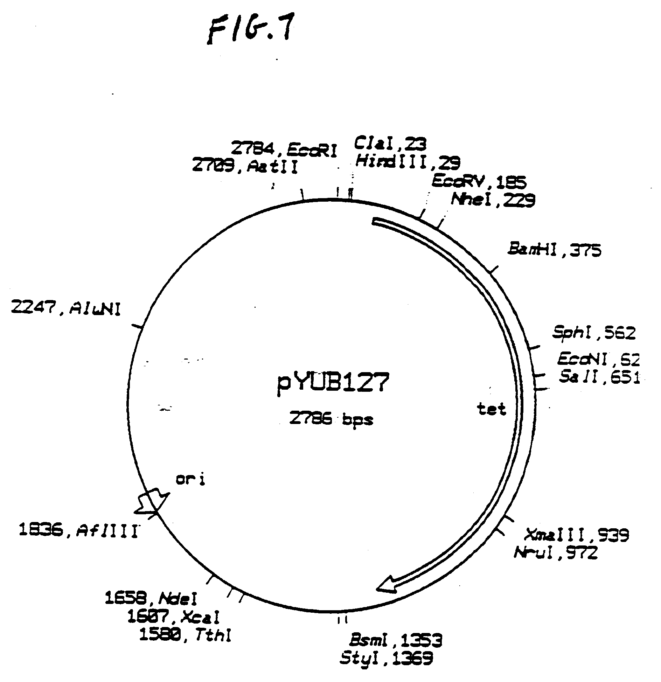

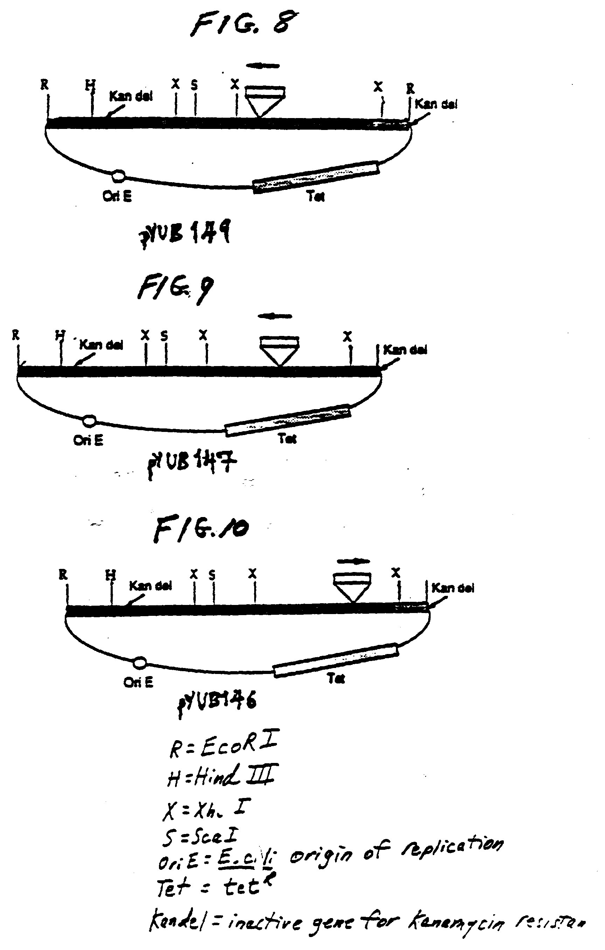

Plasmids pYUB121.24a (Figure 4), pYUB121.3 (Figure 5), and pYUB121.2 (Figure 6) were isolated from methionine auxotrophic mutants of mc²311 according to the procedure of Snapper, et al. (1988). Each of pYUB121.24a, pYUB121.3, and pYUB121.2 was digested with EcoRI. The EcoRI fragments containing Tn5seq1 from each plasmid were subcloned into EcoRI digested pYUB127 (Figure 7), an E. coli vector incapable of replicating in mycobacteria, to obtain the corresponding plasmids pYUB149 (Figure 8), pYUB147 (Figure 9), and pYUB146 (Figure 10), respectively.

BCG-Pasteur was electroporated as described in Bernardini, et al. Proc. Natl. Acad. Sci., Vol. 86, pgs. 3867-3871 (1989) with pYUB146, but with the following modifications. BCG cultures were subcultured 1:50 in 50 ml MADC-TW broth (Snapper, et al., Proc. Natl. Acad. Sci., Vol. 85, pgs. 6987-6991 (1988)) and grown for 10 days at 37°C. The harvested culture was washed first with 50 ml and then with 25 ml of cold glycerol. Following centrifugation, the final pellet was resuspended in 2.5 ml of cold 10% glycerol and 0.4 ml used for each electroporation. BCG transformants were plated on Middlebrook 7H10 agar supplemented with albumin-dextrose complex (ADC), 0.2% glycerol, and 10 µg/ml cyclohexamide containing kanamycin (20 µg/ml) and methionine (50 µg/ml), as described in Snapper, et al. (1988).

It was expected that homologous recombination resulting from a double crossover event, of a linear DNA fragment containing the Tn5 seq 1-inactivated methionine gene would yield kanamycin resistant, methionine auxotrophs of BCG. Unexpectedly, only one methionine auxotroph of over 200 kanamycin-resistant transformants of BCG-Pasteur was obtained from the transformation of linearized pYUB146 containing a Tn5 seq 1-inactivated methionine gene. The transformants were screened according to the procedure of Davis, et al., (1980). Such a result indicates that a high degree of illegitimate, or random recombination has occurred in the transformed BCG-Pasteur organisms in that the Tn5 seq 1 inactivated met gene did not recombine, or integrate, into the homologous met site in the BCG chromosome.

### EXAMPLE 3

### Demonstration of illegitimate recombination in BCG

Total chromosomal DNA from the BCG methionine auxotroph obtained in Example 2, sometimes hereinafter referred to as BCG strain mc² 576, deposited as ATCC No. 55202, and from two other kanamycin-resistant transformants was subjected to Southern blot analysis using the 1.8 kb XhoI DNA fragment from the BCG met gene as a probe. (Figure 11). This probe detects an 8.5 kb fragment in the wild-type BCG chromosome, as shown in Lane 1 of Figure 11. If homologous recombination had taken place, a double cross-over between the chromosome and the linear DNA from the transforming plasmid should result in the replacement of the chromosomal met gene by the insertionally inactivated gene. In Southern blot analysis, the 8.5 kb XhoI fragment of the chromosome should be replaced by two new XhoI fragments, due to the presence of an XhoI site in Tn5 seq 1. The total length of the two fragments should be 8.5 kb plus 3.2 kb, with 3.2 kb being the size of Tn5 seq 1. As shown in Figure 11, however, the auxotroph, mc²576 (lane 2), as well the two other kanamycin-resistant transformants (lanes 3 and 4), contained three XhoI fragments, one 8.5 kb fragment and two fragments A and B, which are identical to that present in the Tn5 seq 1 inactivated BCG met clone. These results indicated that no double cross-over occurred either in the methionine auxotroph, mc² 576 or the two other kanamycin-resistant transformants. Therefore, it is likely that the linear DNA fragment containing the Tn5seq 1 inactivated met gene has integrated illegitimately into the BCG chromosome.

Southern analysis was also performed using Hind III digested chromosomal DNA and the same XhoI met probe. Analysis of wild type BCG detects only one fragment (Figure 11, lane 5). As shown in Figure 11, the internal Hind III fragment C of the donor (plasmid) DNA is conserved in all three clones (lanes 6, 7 and 8). Southern analysis of Hind III digested chromosomal DNA with the probe also detects a flanking fragment D, indicated by an asterisk (*) in the auxotroph (lane 6), and the prototrophs (lanes 7 and 8), the size of which depends upon the site of integration. The variation in size of the flanking fragment D from different BCG recombinants again indicates that the donor, or transforming, DNA fragment is integrating randomly into the BCG chromosome. Also, the Southern blots of XhoI and Hind III digested chromosomal DNA indicated that the position of Tn5 seq 1 in the met gene of the donor was unaltered, and that Tn5 seq 1 did not transpose into the BCG chromosome.

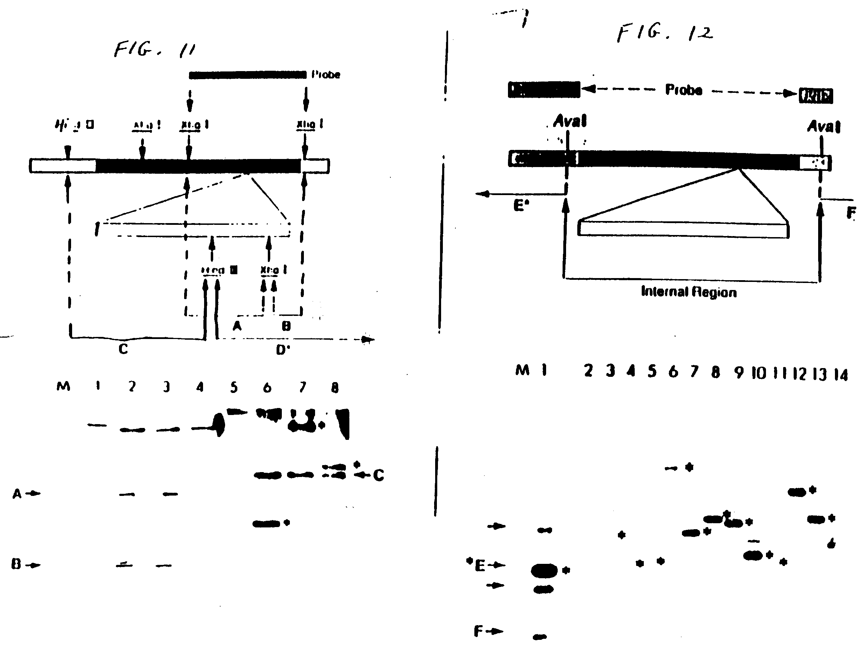

Southern analysis was then carried out upon Hind III digested and XhoI digested chromosomal BCG DNA from nine additional kanamycin-resistance transformants, obtained by the transformation of linearized pYUB146, with the XhoI probe obtained from the BCG met gene. The three donor bands A, B, and C were detected in all nine clones, indicating that homologous recombination had not occurred. Because guanine and cytosine nucleotide rich mycobacterial DNA may yield very large Hind III fragments which are not resolvable by standard gel-electrophoresis, Southern blot analysis was performed by hybridizing Ava I digested chromosomal DNA of the twelve clones with a vector probe, in order to establish the randomness of the integration of band D hereinabove mentioned. As shown in Figure 12, the probe detected four fragments in pYUB146, (lane 1), no fragments in wild type BCG (lane 2) and two internal fragments which were conserved in each clone (lanes 3-14), and fragments E and F, which varied in size and showed different patterns in each clone, indicating that integration had taken place at random sites, and thus establishing that a high degree of illegitimate recombination occurs in BCG.

### EXAMPLE 4

In this example, experiments were undertaken to determine if the illegitimate, or non-homologous recombination hereinabove mentioned is a feature of BCG, or of the methionine (met) gene.

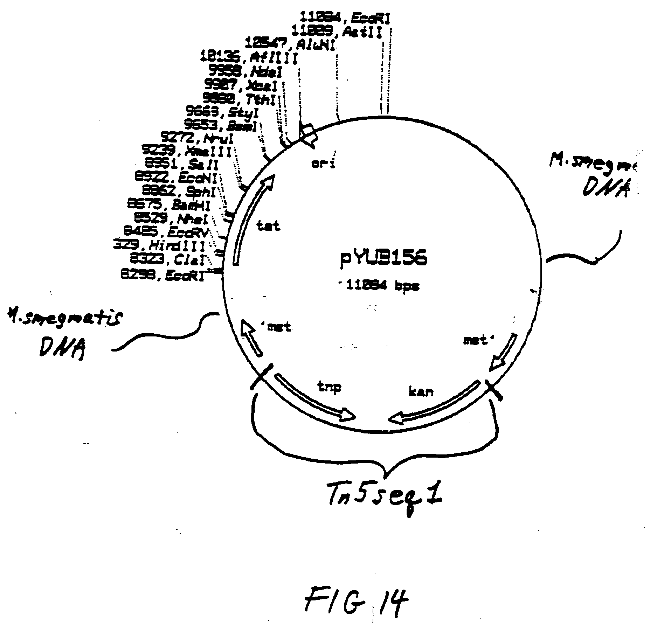

Tn5seq1 is inserted into pBR322 to form pYUB244 (Figure 13). Equal quantities of circular and linearized pYUB244 were electroporated into M. smegmatis. Such electroporation resulted in no integration of either circular or linear pYUB244. Integration was determined by selection for kanamycin resistance (Snapper, et al., (1988)), followed by Southern blot analysis. Equal quantities of circular and linearized pYUB156 (Figure 14), which has Tn5seq1 inserted in the complementing met clone of M. smegmatis, were then electroporated into M. smegmatis. The frequency of kanamycin-resistant colonies using both linear or circular pYUB156 was about equal, as determined by the procedure of Snapper, et al. (1988) followed by Southern blot analysis. Southern blot analysis of six of the kanamycin-resistant colonies of M. smegmatis indicated that integration of pYUB156 occurred at the Eco RI fragment of the chromosome in all six colonies. Such results indicate that mycobacterial homologous sequences are required for integration into the M. smegmatis chromosome, and that integration in M. smegmatis occurs through homologous recombination.

Equal quantities of circular and Eco RI digested pYUB244 and Tn5seq1 inactivated BCG met clones (pYUB146, pYUB147, and pYUB149) were electroporated into BCG. Linear fragments but not circular plasmids, of the above integrated into the chromosome, at a frequency of about 10⁻⁶ to about 10⁻⁴ µg of DNA, irrespective of whether such fragments contained mycobacterial DNA. Integration is determined by two criteria: (1) that colonies are kanamycin-resistant; and (2) that chromosomal DNA from transformants hybridized with the donor molecule as evidenced by Southern blot analysis as hereinabove described.

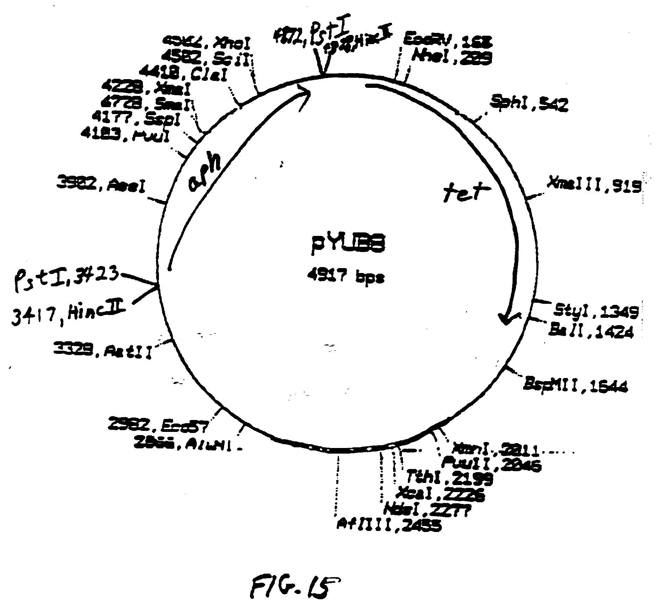

A further experiment was then carried out in which BCG was electroporated with circular and linear pYUB244 and pYUB8 (Figure 15). pYUB8 is a pBR322 derivative is which the amp gene of pBR322 is replaced with the aph (kamamycin resistance) gene of Tn893 (Snapper, 1988), and which lacks sequences from Tn5seq1. In this experiment, circular DNA of both pYUB244 and pYUB8 gave only 1 transformant per ug DNA, whereas linearized and pYUB8 gave 12 and 10 transformants per ug DNA, respectively, according to the procedure of Snapper, et al. (1988), followed by Southern blot analysis. These results indicate that the illegitimate recombination in BCG is not dependent upon the transposase function of Tn5seq1.

### EXAMPLE 5

In this example, equal quantities of circular and linear pYUB244 and pYUB147 were electroporated into the virulent H37Rv strain of M. tuberculosis (obtained from Dr. Wilbur Jones, Centers for Disease Control, Atlanta, Georgia.) The results of such electroporation indicated that the linear forms of both pYUB147 and pYUB244 integrated at a higher frequency into the M. tuberculosis chromosome, as determined by Snapper, et al. (1988), followed by Southern blot analysis. The above results indicate that illegitimate recombination is not restricted to BCG.

### EXAMPLE 6

### Isolation and characterization of BCG isoleucine-leucine-valine auxotrophs using illegitimate recombination.

BCG-Pasteur was transformed via electroporation with linearized pYUB244. Following electroporation, the BCG cells were plated on Middlebrook 7H10 agar containing 0.5mg/ml casamino acids and 20 µg/ml kanamycin. Kanamycin-resistant colonies were screened for their ability to grow on Sauton minimal agar, and were screen for auxotrophy as described in Davis, et al. (1980). Auxonography analysis revealed that one BCG mutant, designated mc²716, required isoleucine, leucine, and valine to grow.

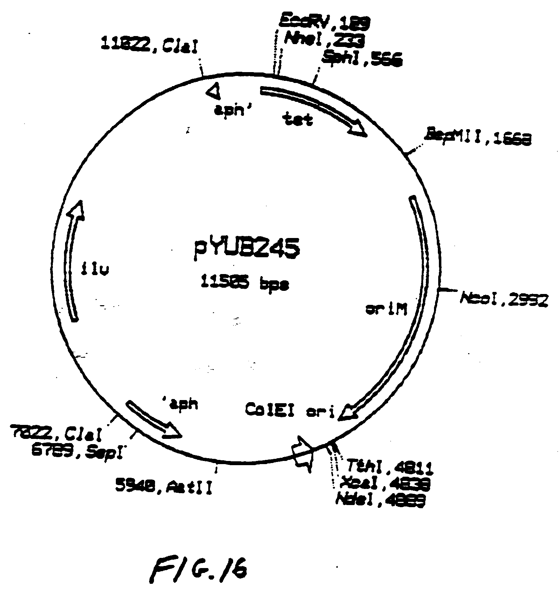

A genomic library of BCG Pasteur was constructed by ligating 4 kb-7 kb Msp-I digested chromosomal fragments to Cla I-digested pYUB53 (Figure 3), as hereinabove described in Example 2. Ligated DNA's were then introduced into E. coli strain ec²270, and plasmids were isolated from the pool of E. coli as described in Snapper, et al. (1988), and were electroporated into BCG strain mc²716, deposited on ATCC No. 55203. A plasmid which conferred prototrophy to mc²716 (referred to as an isoleucine-leucine-valine complementing clone), and which contains The BCG ilv gene, was isolated as described in Snapper, et al. (1988). This plasmid is referred to as pYUB245 (Figure 16).

### EXAMPLE 7

### A. Construction of plasmid containing mutated asd gene of M. smegmatis

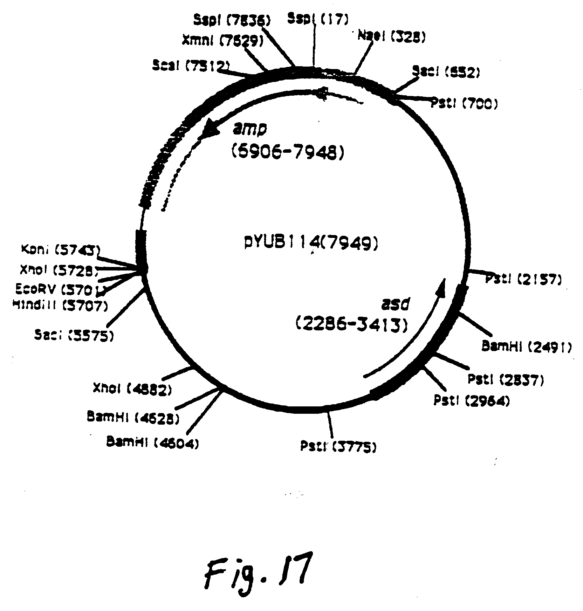

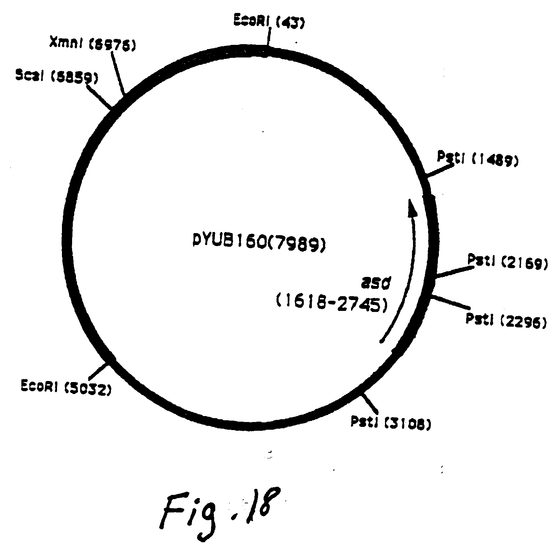

The aspartate semialdehyde dehydrogenase (asd) gene encodes an enzyme that catalyzes an early step in the biosynthesis of diaminopimelic acid (DAP) from aspartate, and thus is a critical component in the cell wall biosynthesis of mycobacteria. A strain containing a mutation in the asd gene and its complementation by the asd gene on a recombinant DNA vector would represent a useful auxotrophic selection cysts for maintenance of vectors in vivo. The M. smegmatis asd gene was cloned by complementing an asd mutant of E. coli and determining the nucleic acid sequence according to the procedure of Jacobs, et al., PNAS, Vol. 83, pgs. 1926-1930 (1986). The asd gene is carried on a 5 kb EcoRI fragment that has two PstI sites in the middle of the gene and two PstI sites flanking the gene. The asd gene was cloned into the EcoRI site of p Bluescript KS + (Stratagene, La Jolla, California), and the resulting plasmid was designated pYUB114. (Figure 17). pYUB114 was digested with EcoRI, the large EcoRI fragment was then isolated and ligated to pGEM7Zf+ (Promega) at the EcoRI site to form pYUB160 (Figure 18). pYUB160 was cut with PstI, and a large fragment having a deletion of the central 1,637 bp of the asd gene was isolated. pYUB8 (Figure 15) was cut with PstI, a PstI kan ^{R}cassette was isolated from pYUB8 and ligated to the large PstI fragment from pYUB160.

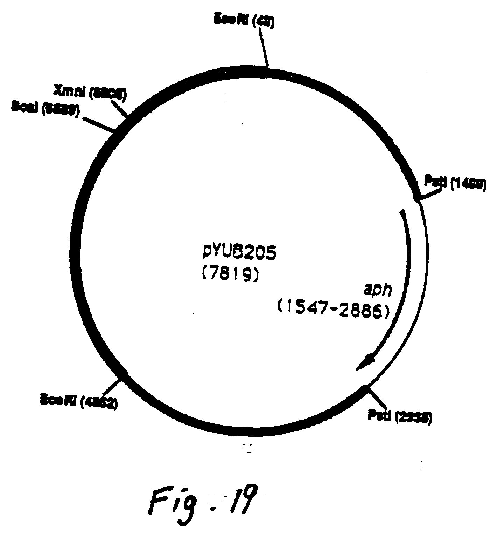

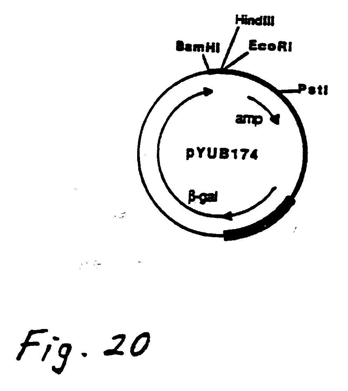

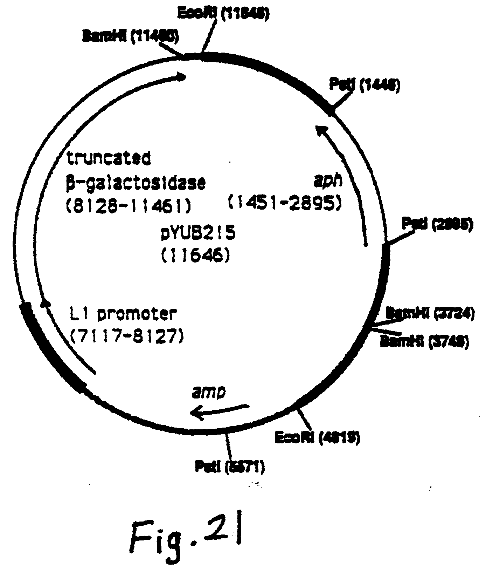

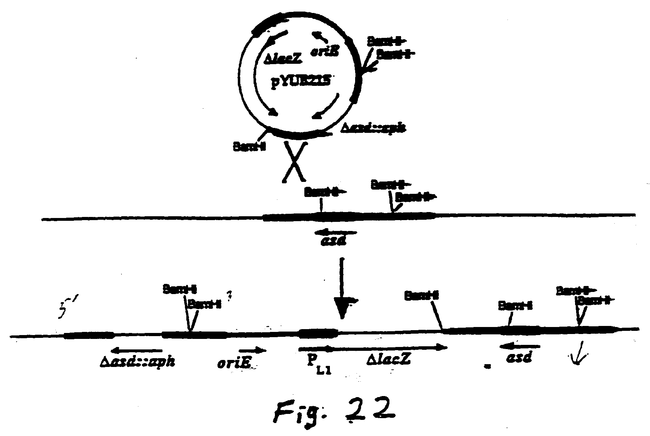

The resulting ligation was transformed into E. coli strain DH5α selecting for ampicillin resistance carried on pYUB114 by electroporation. (Dower, et al., Nucl. Acids Res., Vol. 16, pgs. 6127-6145). Transformants were screened for replacement of the central 1,637 bp of the asd gene with the 1.3 kb kan^{R} cassette. The resulting plasmid, pYUB205 (Figure 19), has a 1,637 bp deletion of the asd gene which is replaced by the aph gene, such deletion and replacement sometimes hereinafter referred to as Δ asd :: aph. pYUB205 then was cut with EcoRI and the large fragment isolated from an agarose gel. The fragment was then ligated to pYUB174 (Figure 20) which had been cut with EcoRI and phosphatased with calf intestinal phosphatase (Boehringer Mannheim, Indianapolis, Indiana). The plasmid pYUB174, which contains the β-galactosidase gene was constructed by placing the previously isolated mycobacteriophage L1 promoter (Barletta, et al., J. Gen. Microbiol., Vol. , pgs. (1991)) upstream of a truncated β-galactosidase gene, to allow expression of β-galactosidase in mycobacteria. The resulting plasmid, pYUB215 (Figure 21), contains the Δ asd :: aph gene and a truncated β-galactosidase gene controlled by a mycobacteriophage L1 promoter.

### B. Construction of mc²687

pYUB215 was transformed by electroporation into M. smegmatis strain mc²6 according to the procedure of Jacobs, et al., Meth. Enzymol., Vol. 204, pgs. 537-555 (1991). This transformation was then plated out on M-ADC plates with 15 g/l bacto-agar (Jacobs, et al., 1991), and containing kanamycin, added at 10 µg/ml and 5-bromo-4-chloro-3-indolyl-β-D-galactoside (X-gal), added at 80 µg/ml, such medium also sometimes hereinafter referred to as M-ADC-KX. The plates were incubated at 37°C until colonies were visible. Blue colonies were picked and screened by Southern blot analysis to establish integration of pYUB215.

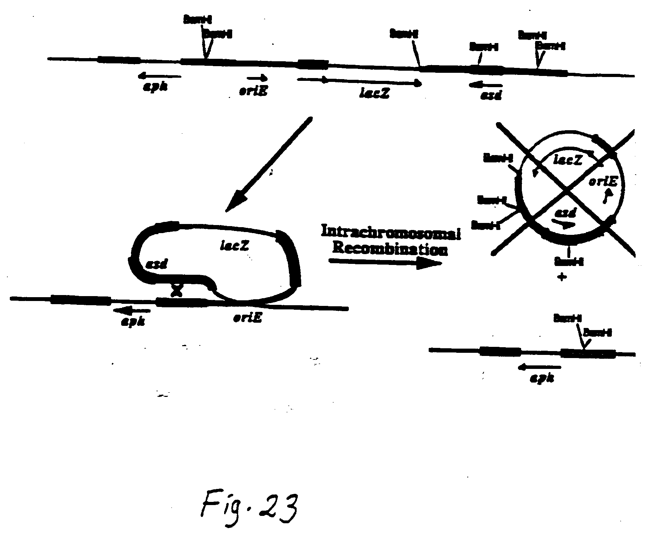

When the plasmid pYUB215 was initially transformed into M. smegmatis, blue and kanamycin resistant colonies were obtained at a frequency of 10⁻⁵ to 10⁻⁶ colonies per microgram of DNA, as compared to a transformation efficiency control vector pMV261 (Stover, at al., Nature, Vol. 351, pgs. 456-460 (1991)), on M-ADC-KX agar containing DAP and casamino acids. No significant numbers of white colonies were obtained above a background of 1 to 5 colonies. The blue colonies were inferred to be generated by a single crossover recombination event between pYUB215 and the M. smegmatis chromosome. The single crossover event likely occurred between the M. smegmatis DNA sequences that flank the aph gene that are identical to the M. smegmatis DNA sequences that flank the asd gene in the M. smegmatis chromosome. Southern analysis of 8 independent transformants confirmed that pYUB215 had integrated on either side of the aph gene, and one such transformant was designated strain mc²687. The inability to generate replacements of the chromosomal asd gene with the mutant Δ asd :: aph allele is interpreted as indicating that the asd gene is essential or that double crossover events occur at an extremely low frequency in mycobacteria. The necessity of having simultaneous double crossover events can be observed by constructing the chromosomal Δ asd :: aph mutant in a two-step process. Because mc²687 has already undergone a single crossover event to integrate pYUB215, a second recombination event between homologous sequences of the two alleles in the chromosome may produce the desired mutant. In the second recombination event, shown in Figure 23, the 3' end of the asd gene aligns itself with the 3' end of the Δ asd :: aph gene. These two "3' end" sequences are homologous. Through intrachromosomal recombination, a circular plasmid, which contains the asd gene, the lac Z gene, and an E. coli origin of replication (ori E) should be looped out of the chromosome. Thus it should be possible to screen for intrachromosomal recombination by the loss of β-galactosidase when the plasmid sequences are looped out during this event because the plasmid does not include a mycobacterial origin of replication.

### C. Isolation of β-galactosidase mutants of mc²687

Strain mc²687 was grown up in M-ADC-TW medium and kanamycin, and aliquots were plated out on M-ADC agar with the addition of kanamycin and X-gal and screened for the production of white colonies.

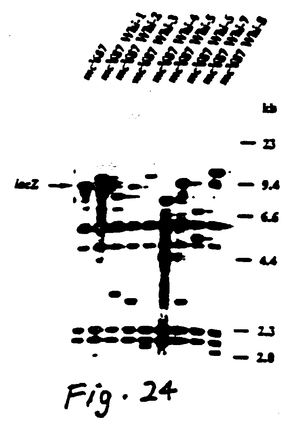

White mutants of mc²687, having lost β-galactosidase activity, were obtained at a frequency of 8 x 10⁻⁵ per cell. Southern analysis of BamHI digests of total chromosomal DNA from 8 clones compared to mc²687 is shown in Figure 24. The DNA was probed with pYUB215. As shown in Figure 24, from the top of the blot, in the mc²687 lane the second and fifth bands are flanking BamHI fragments to the integrated pYUB215 and the first, third, and fourth bands correspond to internal fragments from pYUB215 (first) or the chromosomal asd gene (third and fourth bands). As shown by the arrow, the lacZ gene (β-galactosidase gene) fragment is the largest band, at about 9.5 kb. All white mutants show a shift in the size of the lacZ band and an extra smaller band. The size of the smaller band added to the lacZ band results in a fragment of about 11.5 kb in length, which can be explained by insertion of an approximately 2.2 kb insertional element (IS-element), or transposon, which contains a BamHI site, into the lacZ gene.

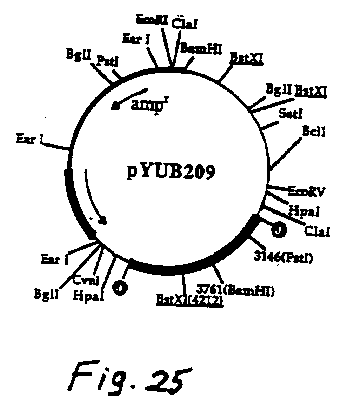

The insertion element which is inserted in the β-galactosidase gene can be recovered by cutting total chromosomal DNA isolated from a clone containing the insertional element with EcoRI to completion. EcoRI digestion will free a fully functional E. coli plasmid because the Δ asd :: aph gene was cloned into pYUB174 at the EcoRI site. Digestion with EcoRI will free the original pYUB174 plasmid which now contains a copy of the insertion element. To isolate the insertion element, EcoRI digested DNA was self-ligated at a DNA concentration of less than 5 ng/µl and transformed into the E. coli strain DH5 , selecting for ampicillin resistance encoded by the β-lactamase gene carried on pYUB174. One clone containing the insertional element, designated as IS1096, was designated as pYUB209 (Figure 25).

### D. Characterization of IS1096

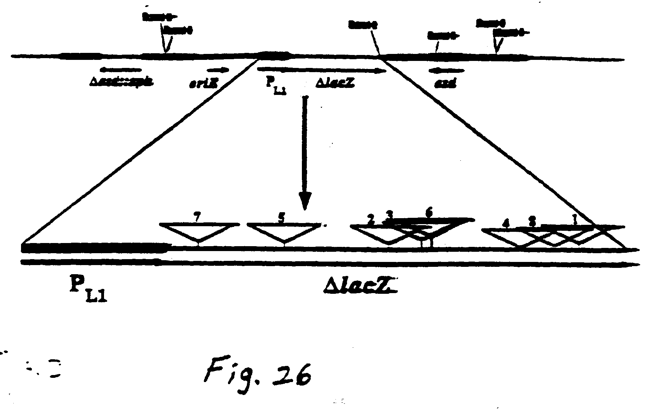

The approximate positions of eight insertions of IS1096 into the β-galactosidase gene are shown in Figure 26. The approximately positions of the eight IS1096 transpositions into the pYUB215 β-galactosidase gene were deterined by Southern analysis. Restriction mapping of IS1096 gave the orientation of the BamHI site within the insertion element to allow accurate approximation of the positions of the insertions into the lacZ gene. From the distribution of insertions in the β-galactosidase gene, IS1096 transposes in a random fashion.

In order to determine more accurately the frequency of IS1096 transpositions in M. smegmatis, 10 individual colonies of mc²687 were grown to stationary phase in 5 ml M-ADC-TW with kanamycin until saturation. Dilutions of these 10 independent cultures were plated out on M-ADC-KX plates such that approximately 1,000 colonies per plate were obtained. The cells from each culture were then plated on 20 M-ADC-KX plates to screen for white colonies. The number of white colonies divided by the total number of colonies present represents the frequency of isolation of mutations in the β-galactosidase gene of mc²687. The average frequency of loss of β-galactosidase activity in these cultures was 7.2 x 10⁻⁵ per cell. This number includes only those colonies which proved to be truly negative for β-galactosidase activity when secondarily screened on X-gal containing plates.

### E. Restriction analysis of IS1096

IS1096 in pYUB209 was located within the β-galactosidase gene between two HpaI sites. To facilitate sequencing of the insertion element, this HpaI fragment was cloned in both orientations into the SmaI site of pGEM7Zf+ (Promega). Once this fragment was cloned into pGEM7Zf+, several deletions were constructed using opportune sites in IS1096 and the polylinker of IS1096. Deletions were made from BstXI and BamHI sites within IS1096 in both directions into the polylinker of pGEM7Zf+. Having these deletions allowed sequencing in both directions from these sites into IS1096. Sequencing of both strands of IS1096 and the junction between IS1096 and pYUB209 was done using synthetic oligonucleotides as well. Sequencing reactions were carried out using Sequenase Version 2.0 (United States Biochemical Corp.) on double stranded DNA templates, as described in Kraft, et al., Bio Techniques, Vol. 6, pqs. 544-547 (1988).

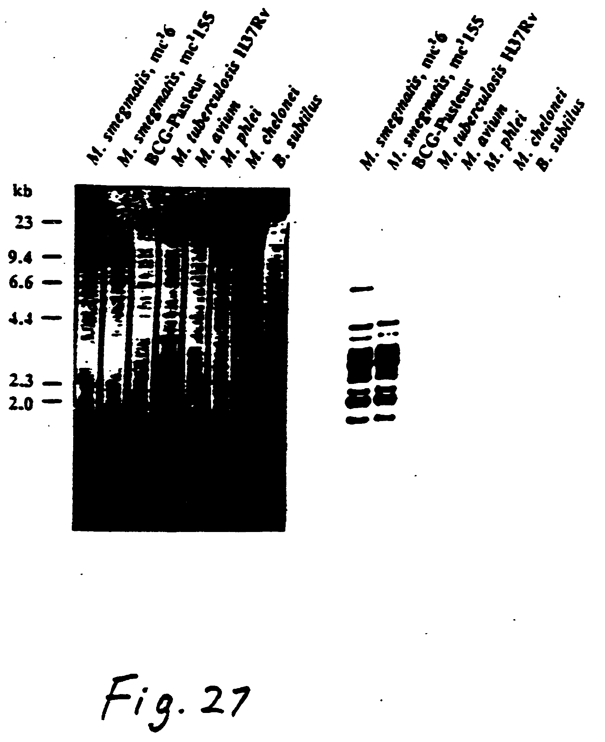

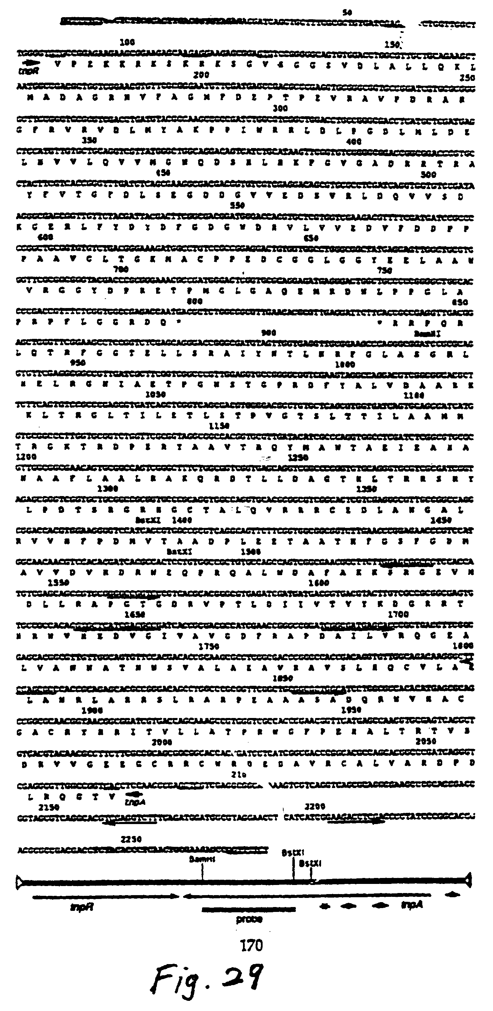

Southern analysis of Streptomyces coelicolor, E. coli strain K-12, Bacillus subtilus, and 12 species of mycobacteria was performed using the internal IS1096 fragment, shown as a solid bar between the BamHI and BstXI sites shown as a graphic representation in Figure 29, as a probe. As shown in Figure 27, IS1096 is present only in M. smegmatis. The pathogenic strains of mycobacteria, M. tuberculosis, M. bovis, M. avium, and M. leprae, lack the insertion element.

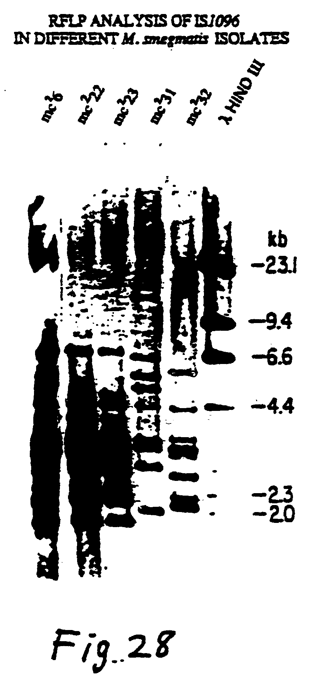

Several different M. smegmatis isolates, including the three morphotypes of M. smegmatis, strain ATCC607, were probed by Southern analysis to determine the degree of variation in restriction pattern when probed with IS1096. Southern analysis was performed with IS1096 upon PstI digests of total chromosomal DNA isolated from M. smegmatis mc²6, mc²22, mc²23, mc²31, mc²32, and DNA cut with HindIII (size standard radioactively labeled). The blot is shown in Figure 28. Lane 1 is mc²6, lane 2 is mc²22, lane 3 is mc²23, lane 4 is mc²31, lane 5 is mc²32, and lane 6 is λ DNA cut with HindIII size standard radioactively labeled. As shown in Figure 29, there is considerable variability in the different isolates. Only one band appears in all of the isolates, and the number of insertional elements found ranges from 8 to 16. Both mc²22 and mc²23 were found to have an additional copy of IS1096 not present in mc²6.

### F. Nucleotide sequence of IS1096

The sequence of IS1096 is shown in Figure 29. Sequence analysis, carried out as hereinabove described, revealed that IS1096 is 2275 bp in length, and the percentage of guanine and cytosine (G+C) is 67%, which is equal to that normally observed in the M. smegmatis chromosome. (Wayne, et al., J. Bacteriol., Vol. 96, pgs. 1915-1919 (1968)). The internal regions of IS1096 have areas of higher G+C ranging from about 70% to about 80%, as well as areas similar to the average G+C content; however, the G+C content of the ends of the insertional element have a G+C content of less than 60%.

A well conserved inverted repeat sequence is observed at both ends of IS1096, which is 25 bp in length, is indicated in bold type in Figure 29. Two mismatches are present in the inverted repeats. Transposition into the β-galactosidase gene resulted in a duplication of 8 bp, indicated by underlined bold type, of target DNA sequences on both sides of the insertion point. This duplication is consistent with the mechanism of transposition of most insertion elements. Three sets of inverted repeats of approximately 9 bp in length were found in the 3' end of IS1096, the positions of which are shown in Figure 29, as designated by pairs of arrows in opposite orientation.

Open reading frame (ORF) analysis of the sequence of IS1096 revealed the presence of 13 ORF's which are longer than 100 amino acids. None of the putative ORF's displayed high levels of homology to proteins encoded by other insertion elements; however, two of the ORF's may be distantly related to previously sequenced tnp A transposase and tnp R genes, as described in Turner, et al., Nucl. Acids Research, Vol. 17. pg. 1757 (1989) and in Rowland, et al., Mol. Microbiol., Vol. 4, pgs. 961-975 (1990), as shown in Figure 29. A large 414 amino acid ORF with a valine imitation codon was found which has 21.1% identical and 66% homologous amino acids over 185 amino acids with the transposase from Tn 3926 (Turner, et al., (1989)). A 237 amino acid open reading frame which also begins with a valine is in the opposite direction to the putative tnp A gene as shown in Figure 29 and designated as tnp R. The amino terminal 92 amino acids of tnp R are 18.5% identical and 69% homologous to the amino terminus of the Tn1000 resolvase. (Reed, et al., Nature, Vol. 300, pgs. 381-383 (1982)). The proteins encoded by these ORF's are of a similar size and significantly more similar than the other ORF's found on IS1096 to standard transposes and resolvase proteins. This may indicate that such ORF's encode proteins which are involved in the transposition of IS1096, but are distantly related to well-characterized proteins known to be involved in the transposition of other transposons.

### Example 8

IS1096 is cloned out of M. smegmatis, and a construct is made in which a sequence thought to encode transposase(s) was removed from IS1096 and replaced with an aph gene (which was hereinabove described). The sequence thought to encode transposase(s) remains in the resulting construct but is located outside of the inverted repeat sequences. The resulting construct is then cloned into pGEM7Zf+. The resulting plasmid is then transformed into M. bovis-BCG, and M.bovis-BCG cells are plated out on complete nutrient medium containing kanamycin and selected for kanamycin resistance. Surviving colonies are those in which the transposable element has transposed from the plasmid into the BCG chromosome. The transposes gene(s) remains in the plasmid, which cannot replicate in mycobacteria. The plasmid, therefore, will become lost. Because the transposase gene(s), which remains in the plasmid, will become lost, the transposable element will remain in the region of the BCG chromosome into which it inserted, and will not transpose into another region of the chromosome or into any other expression vehicles which may also be present in the cell.

Upon selection of surviving colonies, the colonies may be screened for specific mutations by plating on various media.

It is to be understood, however, that the scope of the present invention is not to be limited to the specific embodiments described above. The invention may be practiced other than as particularly described and still be within the scope of the accompanying claims.

## Claims

1. A recombinant mycobacterium comprising at least one mycobacterial gene containing an insertional mutation.

2. The mycobacterium of claim 1, wherein the mycobacterial gene is a gene that encodes an enzyme that is essential in the biosynthetic pathway of a nutrient or an amino acid.

3. The mycobacterium of claim 1, wherein the insertional mutation is effected by homologous recombination.

4. The mycobacterium of claim 1, wherein the insertional mutation is effected by illegitimate recombination.

5. The mycobacterium of claim 1, wherein the insertional mutation is effected by a transposon.

6. The mycobacterium of claim 1, which is selected from the group consisting of M. tuberculosis, M. bovis-BCG, M. avium und M. leprae.

7. A method for preparing a recombinant mycobacterium comprising introducing an insertional mutation into at least one mycobacterial gene of a mycobacterium.

8. The method of claim 7, wherein the mycobacterial gene is a gene that encodes an enzyme that is essential in the biosynthetic pathway of a nutrient or an amino acid.

9. The method of claim 7, wherein the insertional mutation is effected by homologous recombination.

10. The method of claim 7, wherein the insertional mutation is effected by illegitimate recombination.

11. The method of claim 7, wherein the insertional mutation is effected by a transposon.

12. The method of claim 7, wherein the mycobacterium is selected from the group consisting of M. tuberculosis, M. bovis-BCG, M. avium and M. leprae.
